# EUROPEAN PATENT SPECIFICATION

(11) **EP 1 689 521 B1**
(45) Date of publication and mention of the grant of the patent: **05.10.2016**
(21) Application number: 03819054.2
(22) Date of filing: 24.11.2003
(51) Int. Cl.: B01J 20/18, C07C 29/76, C01B 39/02

(54) **PREPARATION OF MOLECULAR SIEVE USED FOR THE DEHYDRATION OF THE ALCOHOL**
HERSTELLUNG VON MOLSIEB, DAS ZUR DEHYDRATISIERUNG VON ALKOHOL VERWENDET WIRD
PREPARATION D'UN TAMIS MOLECULAIRE UTILISABLE DANS LA DESHYDRATATION DES ALCOOLS

(43) Date of publication of application: 16.08.2006
(73) Proprietor: Council of Scientific and Industrial Research, New Delhi 110 001 (IN)
(72) Inventor: JASRA, Raksh, Vir, Bhavnagar 364 002 Gujarat (IN); SEBASTIAN, Jince, Bhavnagar 364 002 Gujarat (IN); CHUDASAMA, Chintansinh, D., Bhavnagar 364 002 Gujarat (IN)
(74) Representative: Hirsch & Associés
(86) International application number: PCT/IB2003/005324
(87) International publication number: WO 2005/051533

(56) References cited:
- EP-A- 0 049 936
- US-A- 4 933 162
- US-A- 5 130 166
- PATENT ABSTRACTS OF JAPAN vol. 014, no. 382 (C-0749), 17 August 1990 (1990-08-17) & JP 02 139034 A (RES DEV CORP OF JAPAN), 29 May 1990 (1990-05-29)
- DATABASE WPI Section Ch, Week 198726 Derwent Publications Ltd., London, GB; Class H04, AN 1987-183291 XP002275448 & JP 62 115088 A (KEISHITSU RYUBUN SHINYOTO), 26 May 1987 (1987-05-26)
- PATENT ABSTRACTS OF JAPAN vol. 013, no. 024 (C-561), 19 January 1989 (1989-01-19) & JP 63 230515 A (JGC CORP), 27 September 1988 (1988-09-27)

## Description

### Field of the invention

The present invention relates to a process for the preparation of a molecular sieve adsorbent for the adsorptive dehydration of alcohols.

The invention relates to the preparation and use of surface modified zeolites (activated molecular sieve) in the dehydration of alcohols. More specifically, the invention relates to the preparation and use of a molecular sieve adsorbent, which selectively removes water from water - ethanol azeotrope obtained from distillation of the crude synthetic or fermentation feedstock.

### Background and prior art

The use of anhydrous alcohol (99.5 vol. % ethanol) has become an important consideration as a means of saving gasoline produced from high-cost crude oil. It is a well-established fact.that up to 20 percent anhydrous ethanol can be blended with gasoline to obtain a relatively high-octane antiknock fuel, which can be used for internal combustion engines. With some engine modification, anhydrous ethanol can be used as the fuel directly.

Alcohol/water mixtures, such as those produced by fermentation of biomass material form a single liquid phase, which usually contains more or less equal volumes of ethanol and water, at least after initial distillation. Such mixtures are separated conventionally by further distillation, sometimes with addition of benzene, cyclohexane, etc. to yield an anhydrous alcohol fraction, which may contain minor amounts of other alcohols, such as propyl or butyl. Adsorption and solvent extraction are alternative or supplemental methods of separating alcohol and water. An increasing use of alcohol is seen for fuel, often in admixture with fossil fuels, such as gasoline or even diesel oil, for example, in which anhydrous conditions are favoured.

Over the past 30 years a series of distillation systems have been developed for the efficient recovery of ethanol from synthetic and fermentation feedstock. These units produce high-grade industrial alcohol, anhydrous alcohol, alcoholic spirits, and ethanol for motor fuels. Ethanol quality and recovery have been improved; at the same time energy consumption has decreased.

Synthetic ethanol is purified in a simple three-column distillation unit where in the recovery is 98 %, and the high-grade product contains less than 20 mg/kg of total impurities and has a permanganate time of over 60 min.

The following are the key features for the efficient recovery of high-grade ALCOHOL especially ethanol from fermentation feed stocks:
1) *Extractive distillation* results in a higher degree of purity than is possible in conventional purification columns; both investment and operating costs are reduced.
2) *Pressure-cascading installations* and *heat pumps* permit substantial heat recovery and recycling, thus minimizing heat loss and steam consumption. Virtually all (95 - 99%) the ethanol in the crude feed is recovered as high-grade product.
3) *Advanced control systems* ensure stable operating conditions. Product quality can be maintained with a total impurity content of less than 50 mg/kg and a permanganate time of over 45 min.
4) *Energy requirements* are minimized. The flash heat recovered from the grain-cooking system is used to heat the ethanol distillation unit, thus reducing the energy consumption for ethanol production by ca. 10%. Use of a vapour recompression technique can reduce the energy required for the evaporation of stillage to as little as one-tenth of that required in a triple- or quadruple-effect evaporator.

With the ready availability of 95% alcohol through distillation, it might be expected that obtaining 100% (water free) alcohol would provide little problem. However, this is not the case, for no matter how efficient or long the distillation process, 95% alcohol or any lower-strength solution cannot be further concentrated beyond about a 96.4% alcohol solution by weight under standard conditions. At approximately that point, equilibrium is reached in which the liquid and vapour mixtures have the same composition. This is called an azeotrope or a constant-boiling mixture. In the case of ethyl alcohol, this is a binary azeotrope of the minimum-boiling variety. It has been reported that pressure changes affect this azeotropic mixture.

To produce anhydrous ethanol, the water - ethanol azeotrope obtained from distillation of the crude synthetic or fermentation feedstock must be dehydrated. For economic reasons, large distilleries rely mostly on azeotropic distillation for ethanol dehydration. Benzene has been used as an azeotropic dehydrating (entraining) agent in many plants, but some concern exists about its carcinogenicity and toxicity. Cyclohexane and ethylene glycol are used in some distilleries as effective dehydrating agents.

Some smaller ethanol plants use molecular sieve adsorption techniques to dry the ethanol azeotrope. Pervaporation through semipermeable membranes or use of a solid dehydrating agent may reduce energy and equipment costs.

Growing requirements for anhydrous ethanol for use in motor fuel gasoline blends require systems that operate with a minimum of energy and that are also reliable in continuous operation. Although production and blending of ethanol with gasoline have been practiced in different countries during the past forty years, the use of ethanol in such blends has been limited because of the relatively high costs of production.

The conventional distillation system for recovering motor fuel grade anhydrous ethanol from a dilute feedstock, such as fermented beer or synthetic crude alcohol, utilizes the three essential steps, (i) stripping and rectifying operation; (ii) dehydration and (iii) condensation and decantation in three different towers. In the first tower the feedstock containing, 6 to 10 vol.% ethanol is subjected to a preliminary stripping and rectifying operation in which the concentration of water is materially reduced and concentrated ethanol stream is removed which contains in the order of 95 vol.% ethanol, thereby approaching the ethanol-water azeotrope composition of about 97 vol.% ethanol. The concentrated ethanol stream is next subjected to azeotropic distillation in the second or dehydrating tower using a suitable azeotropic or entraining agent, usually benzene or a benzene-heptane mixture. This results in removal of most of the remaining water, and the desired motor fuel grade anhydrous ethanol product (99.5 vol. %) is recovered from the dehydrating tower. The third tower of the system comprises a stripping tower in which the benzene or other azeotropic agent is recovered from the water-rich phase following condensation and decantation of the azeotropic overhead stream from the dehydrating tower.

One of the key elements in the high operating cost of the above described conventional distillation system, is the high thermal energy requirement of the system, particularly steam consumption. The conventional system also has other serious shortcomings, which detract from the commercial feasibility of the use of anhydrous ethanol as motor fuel. For example, the stripper-rectifier tower is occasionally operated under super atmospheric pressure, which results in higher temperatures, which in turn cause rapid fouling and plugging of the trays. As a consequence, periodic interruption of the operation is necessary to permit cleaning of the tower with resultant high maintenance costs. Furthermore, the conventional system does not include adequate provision to overcome the operating difficulties and product quality problems caused by the presence of higher boiling and lower boiling impurities in the feedstock.

In the prior art, to satisfy the ever-growing demand for absolute alcohol on a commercial scale, several continuous methods have been used. The first was based on a patent issued to Donald B. Keyes, U.S. Pat. No. 1,830,469 relies upon the dehydration of ethyl alcohol by the formation of a ternary azeotrope with benzene, ethyl alcohol and the remaining water in a 95% alcohol solution. This azeotropic mixture, having a low boiling point, is distilled off and must be separated by further secondary operations, leaving anhydrous ethyl alcohol at the bottom of the rectification column. Many other compounds have been suggested for use in similar azeotropic distillations, including ethyl ether, methylene chloride, isobutylene, isooctane, gasoline, benzene and naphtha, isopropyl ether, methyl alcohol and acetone. All of these distillations suffer from similar problems, however, those being increased cost and increased danger from fire or explosion during processing due to the added components.

A second process, based on the patent to Joseph Van Ruymbeke, U.S. Pat. No. 1,459,699 relies upon a reflux of glycerine in the column to act as a dehydrating agent. The glycerine and water pass out at the bottom of the still with the distillate being anhydrous ethyl alcohol. Considerable alcohol is caught up with the glycerine and water, however, and must be recovered in a second rectifying still. Yet another method, reported to be the earliest of its kind, utilizes anhydrous potassium carbonate as the drying agent. Many other inorganic compounds have been similarly studied, such as calcium oxide, calcium carbide, calcium sulphate, calcium aluminium oxide, aluminium and mercuric chloride, zinc chloride and sodium hydroxide, some of which are suggested as additives in the glycerine refluxing process mentioned above. The limitation of this processes are that its required two-step rectifying column and in another additive inorganic materials are not eco-friendly.

U.S. Pat. No. 4,161,429 (1979) to J. J. Baiel, et al. discloses a high-pressure (100 - 200 Psi) azeotropic distillation process of ethanol conducted in the absence of oxygen using pentanes and cyclohexane as entrainers. The drawbacks associated with the process are (i) it requires high-pressure distillation and (ii) continuously maintaining the oxygen free atmosphere is difficult.

U.S. Pat. No. 4,217,178 (1980) to R. Katzen, et al. discloses an improved distillation method for obtaining motor fuel grade anhydrous ethanol from fermentation or synthetic feedstock. The three-tower system used in the anhydrous ethanol production comprising a stripper-rectifier tower in which the dilute feedstock is converted to a concentrated ethanol stream, a dehydrating tower in which water is removed from the concentrated ethanol stream by azeotropic distillation, and a stripper tower for recovering the azeotropic agent. The limitation of this process is the high operating pressure and the complete removal of the aziotropic agent from the anhydrous ethanol is difficult.

U.S. Pat. No. 4,256,541 (1981) to W. C. Muller, et al. discloses a method for distillation of anhydrous (absolute) ethanol with high thermal efficiency from any dilute feedstock using cyclohexane as the azeotrope-forming agent. The limitation of the process is that the process involves the use of cyclohexane as aziotropic forming agent during the aziotropic distillation.

U.S. Pat. No. 4,273,621 (1981) to L. L. Fornoff discloses a process for dehydrating aqueous ethanol, utilizing a high-pressure distillation with a single distillation column of an aqueous ethanol admixture, to achieve a vapour phase ethanol-water admixture containing about 90 %, by weight, of ethanol, and then drying the vaporous admixture, in the presence of CO₂, with a crystalline zeolite type 3A. The limitation of the process is that the low water adsorption capacity and low hydrothermal stability of the zeolite 3A type adsorbent.

U.S. Pat. No. 4,277,635 (1981) to C. S. Oulman, et al. discloses a process for concentrating relatively dilute aqueous solutions of ethanol by passing through a bed of a crystalline silica polymorph, such as silicalite, to adsorb the ethanol with residual dilute feed in contact with the bed, which is displaced by passing concentrated aqueous ethanol through the bed without displacing the adsorbed ethanol. A product concentrate is then obtained by removing the adsorbed ethanol from the bed together with at least a portion of the concentrated aqueous ethanol used as the displacer liquid. The limitation of the process is the process requires passing of concentrated ethanol for the recovery of the anhydrous ethanol.

U.S. Pat. No. 4,301,312 (1981) to H. M. Feder, et al. discloses a process for the production of anhydrous ethanol by using a transition metal carbonyl and a tertiary amine as a homogeneous catalytic system in methanol or a less volatile solvent to react methanol with carbon monoxide and hydrogen gas producing ethanol and carbon dioxide. The gas contains a high carbon monoxide to hydrogen ratio as is present in a typical gasifier product. The reaction has potential for anhydrous ethanol production as carbon dioxide rather than water is produced. The only other significant by product is methane. The drawbacks of the process are that it involves the use of inflammable hydrogen and carbon monoxide and the formation of by product methane.

U.S. Pat. No. 4,306,884 (1981) to E. R. Roth discloses a process for the separation of alcohol/water mixtures by extraction of alcohol with a solvent especially suited to such extraction and subsequent removal with addition of gasoline between the solvent extraction and solvent recovery steps. The limitation of the process is that it can produce only denatured ethanol, which contains the solvents used for the extraction of ethanol.

U.S. Pat. No. 4,306,940 (1981) to S. Zenty discloses a process and apparatus especially suited for distilling alcohol from aqueous fermentation liquors wherein liquid vapours from a liquid mixture is pre heated with the product. The limitation of the process is that it can produce only a water-ethanol aziotropic mixture containing about 95% of ethanol. The production of anhydrous ethanol requires additional purification steps.

U.S. Pat. No.4,306,942 (1981) to B. F. Brush, et al. discloses an improved distillation method and apparatus are provided for recovering hydrous ethanol from fermentation or synthetic feedstock with a multiple heat exchange steps. The limitation of the process is that it can produce only a water-ethanol aziotropic mixture containing about 95% of ethanol. Anhydrous ethanol production requires additional dehydration steps.

U.S. Pat. No. 4,308,106 (1981) to R. L. Mannfeld revels a process and still for removing substantially all water from an alcohol-containing solution using a rectification column under reduced pressure of about 40mmHg or less to get alcohol having a water content of about 2% by volume or less. The limitation of the process is that maintaining very low pressure for the distillation, is difficult and needs specially designed pumps.

U.S. Pat. No. 4,346,241 (1982) to J. Feldman provides a process for obtaining substantially anhydrous ethanol from a dilute aqueous ethanol solution in which the ethanol stream is subjected to liquid-liquid extraction to provide an ethanol-poor raffinate phase and an ethanol-rich extract phase, the ethanol present in said latter phase is concentrated in a rectifying column to provide an aqueous ethanol of high proof and the concentrated ethanol is azeotropically distilled in an anhydrous column operated under substantially super atmospheric pressure at high temperature. The drawbacks associated with the process are the use of amines as the extactant for the extraction of ethanol. Also, multi steps are involved which increases the unit operation as well as the time for dehydration.

U.S. Pat. No. 4,349,416 (1982) to H. S. Brandt, et al. discloses a process and apparatus for the separation of components from a mixture, which forms an azeotrope, by subjecting the mixture to extractive distillation to remove one of the components and regeneration to separate another component from the extracting agent added to the extractive distillation column. The drawbacks associated with the process are the use of aziotropic forming agents and the extractive distillation process involved in the separation.

U.S. Pat. No. 4,351,732 (1982) to J. D. Psaras, et al. provides a process and apparatus for dehydrating liquid phase ethanol in an adsorber unit containing at least two towers that cycle between adsorption and desorption cycles, characterized in the desorption cycle by an indirect heating volatilisation of absorbed and adsorbed liquid at ambient pressures, and by a final stages desorption under sub-atmospheric pressures. The limitation of the process is that the low water adsorption selectivity and capacity of the adsorbent.

U.S. Pat. No. 4,366,032 (1982) to P. Mikitenko, et al. provides a process for dehydrating aliphatic alcohols water mixture wherein the alcohols-water mixture is subjected to a first fractionation in the presence of a selective solvent, giving a vapour effluent containing dehydrated light alcohols and a liquid phase containing heavy alcohols, water and the selective solvent, said liquid phase being subjected to a second fractionation giving as vapour effluent an hetero-azeotropic mixture of water and heavy alcohols and, a liquid effluent. The limitation of the process is the use of aziotropic forming agents and the extractive distillation process involved in distillation process.

U.S. Pat. No. 4,372,822 (1983) to W. C. Muller, et al. discloses a process for the preparation of anhydrous ethanol by distillation with thermal efficiency from a dilute feedstock. The columns are operated at substantially super atmospheric pressure with thermal values recovered from these columns being used in the operation of the rectifying column. The limitation of the process is that it requires high-pressure and elevated temperature for the anhydrous ethanol production.

U.S. Pat. No.4,422,903 (1983) to J. R. Messick, et al. discloses an improved distillation method and apparatus for recovering anhydrous ethanol from fermentation or synthetic feedstock. The system includes at least one stripper-rectifier tower, a dehydrating tower, and an azeotropic agent stripping tower, at higher pressure than the stripper-rectifier tower and by condensing the overhead vapours from the dehydrating tower. The drawback of the process is that it is a multi stage process at elevated temperature and pressure. It also involves the use of azeotropic forming agents in the distillation process.

U.S. Pat. No.4,428,798 (1984) to D. Zudkevitch, et al. discloses a process for separating low molecular weight alcohols, especially ethanol, from aqueous mixtures. The process involves subjecting alcohol-water mixtures to extraction and/or extractive distillation procedures. Extractive solvents useful for the process of this invention include phenols having at least six carbon atoms and a boiling point between 180°C and 350°C. The limitation of the process is the use of phenols as extractive solvent for the azeotropic distillation process at higher temperature and pressure. Moreover, the removal of phenol from dehydrated ethanol is also essential.

U.S. Pat. No. 4,455,198 (1984) to D. Zudkevitch, et al. discloses a process for ethanol concentration from ethanol-water mixtures by extraction or extractive distillation with a solvent, a cyclic ketone of at least seven carbons or cyclic alcohol of at least eight carbons such a cyclohexylcyclohexanone or cyclohexylcyclohexanol. The preferred solvents are also non-toxic, such that the alcohol can be used for human consumption. The limitation of the process is the use of aziotropic forming agents and the extractive distillation process involved in distillation process.

U.S. Pat. No. 4,559,109 (1985) to F. M. Lee, et al. discloses a process for producing anhydrous ethanol from an ethanol-water mixture feedstock comprising subjecting the feedstock to distillation in a first distillation zone to produce an overhead vapours of from 80 to 90 weight percent ethanol, subjecting the thus produced overhead vapours to extractive distillation in an extractive distillation zone to produce anhydrous ethanol vapour overhead of about 99.5 weight percent ethanol and a solvent-rich bottom stream. The drawback of the process is the azeotropic distillation involves the use of toxic solvents as the azeotrope-forming agent.

U.S. Pat. No. 4,620,857 (1986) to E. Vansant, et al. discloses a process for the porous solid such as a zeolite or clay can be degassed to make it suitable as an adsorbent, after which the entrances of the pores are narrowed to a desired size by treating the porous solid with chemisorbable materials such as diborane. The limitation of the process is that the diborane used for the narrowing of the pores is highly reactive and toxic and the narrowing of the pores may not be uniform.

U.S. Pat. No. 4,645,569 (1987) to T. Akabane, et al. discloses a process for.producing anhydrous ethanol, using an apparatus comprising a combination of a concentration column, an azeotropic distillation column and a solvent recovery column, capable of effectively utilizing the vapour at the top of the concentration column and the azeotropic distillation column. The limitation of the process is that the extractive distillation process consumes very high amount of energy.

U.S. Pat. No. 4,654,123 (1987) to L. Berg, et al. discloses a process for the separation of alcohol/water using extractive distillation in which the water is removed as overhead product and the ethanol and extractive agent as bottoms and subsequently separated by conventional rectification. Typical examples of suitable extractive agents are hexahydrophthalic anhydride; methyl tetrahydrophthalic anhydride and pentanol-1; trimellitic anhydride, ethyl salicylate and resorcinol. The limitation of the process is that the extractive distillation process involves multi steps and involves the use of toxic aziotropic forming agents.

U.S. Pat. No. 4,692,218 (1987) to H. Houben, et al. discloses a method and apparatus for simultaneously producing various forms of alcohol, including ethanol, which can likewise be withdrawn from the apparatus simultaneously. To this end, successive columns in the individual processing stages, each of which includes distillation, rectification, purification and dehydration, are connected in parallel for product flow but in series for energy flow and conservation. The limitation of the process is that it involves different process stages and requires high amount of energy.

U.S. Pat. No. 5,035,776 (1991) to J. P. Knapp discloses a thermally integrated extractive distillation process for recovering anhydrous ethanol from fermentation or synthetic feed stocks with four distillation columns. In the first step the dilute ethanol water mixture is concentrated by distillation. The concentrated ethanol in the first distillation column is then distilled at higher pressures in the second and third distillation column to get the aziotropic mixture of ethanol and water. The aziotropic mixture thus produced is then subjected to extractive distillation to get anhydrous ethanol. The limitation of the process is that it involves multistage distillation and extractive distillation for the production of anhydrous ethanol.

In an approach, chemical vapour deposition technique was used for controlling the pore opening size of the zeolites by the deposition of silicon alkoxide for the size / shape selective separation of molecules [M. Niwa et al., JCS Faraday Trans. I, 1984, 80, 3135-3145; M. Niwa et al., J. Phys. Chem., 1986, 90, 6233-6237; Chemistry Letters, 1989, 441-442; M. Niwa et al., Ind. Eng. Chem. Res., 1991, 30, 38-42; D. Ohayon et al., Applied Catalysis A- General, 2001, 217, 241-251]. Chemical vapour deposition is carried out by taking a requisite quantity of zeolite in a glass reactor, which is thermally activated at 450°C *in situ* under inert gas like nitrogen flow. The vapours of silicon alkoxide are continuously injected into inert gas stream, which carries the vapours to zeolite surface where alkoxide chemically reacts with silanol groups of the zeolite. Once the desired quantity of alkoxide is deposited on the zeolite, sample is heated to 550°C in air for 4-6 hours after which it is brought down to ambient temperature and used for adsorption. The major disadvantages of this technique are (i) Chemical vapour deposition, which leads to non-uniform coating of alkoxide which in turn results in non-uniform pore mouth closure, (ii) The process has to be carried out at elevated temperature where the alkoxide gets vaporised, (iii) The deposition of the alkoxide requires to be done at a slow rate for better diffusion and (iv) This method is expensive and lack of a commercial level at higher scale will be difficult.

To summarize, the known processes are complex, require other additives (such as benzene or ether), which significantly increase cost and potential hazard during use, and fail to provide a safe, efficient, simple method of operation. The applicant's invention described and claimed herein below attempts to meet this need.

### Object of the invention

The main object of the present invention is to provide a process for the preparation of a molecular sieve adsorbent for the adsorptive dehydration of alcohols, which obviates the drawbacks as detailed above.

Still another object of the present invention is to prepare a molecular sieve type adsorbent for the dehydration of the alcohols.

Still another object of the present invention is to provide an adsorbent, which can be prepared by the external surface modification of the microporous solids like zeolites to have molecular sieving effect.

Yet another object of the present invention is to have a uniform deposition of alkoxide on the surface of the zeolites.

Yet another object of the present invention is to prepare a molecular sieve adsorbent with high thermal and hydrothermal stability.

Yet another object of the present invention is to prepare an adsorbent, which selectively adsorbs water from water-alcohol mixtures and can be used commercially for the separation and dehydration of alcohols.

### Summary of the invention

This invention relates to the use of pore mouth control of microporous solids for developing a novel molecular sieve adsorbents and their potential in the drying of alcohols. More specifically, the invention relates to the manufacture and use of a molecular sieve adsorbent, which selectively adsorbs water from the azeotropic alcohol - water mixtures, by pore mouth control of microporous solids with liquid phase metal alkoxide deposition on the external surface at ambient conditions of temperature and pressure. Thus, prepared adsorbent is useful for the commercial drying of alcohols.

### Detailed description of the invention

Accordingly, the present invention provides a process for the preparation of a molecular sieve adsorbent for the adsorptive dehydration of alcohols; the said process comprises the steps of:
a) obtaining a molecular sieve adsorbent represented by the chemical formula, (Na₂O)₆. (Al₂O₃)₆ . (SiO₂)₁₂. (M_{2/n}O₂)ₓ. wH₂O, where M is the element selected from Si, Al, Zr and Ti; n its valance, the values of x varies from 0.001 to 0.1 and w being the number of moles of water;
b) activating the molecular sieve adsorbent at a temperature in the range of 350 to 450 C to eliminate physically adsorbed water, for a period ranging from 3 to 6 hours;
c) cooling the activated the molecular sieve adsorbent under vacuum in the range of 10-2 to 10-4 torr ;
d) treating the activated adsorbent with an alkoxide of element M in a dry solvent;
e) drying the treated activated adsorbent of step (d) in air in static condition at a temperature in the range of 15 to 40 C ;
f) converting the alkoxide of element M deposited on modified adsorbent into metal oxide (M_{2/n}O₂) by calcining the same in a temperature range of 450 to 600 C for a period ranging from 3 to 8 hrs, and
g) obtaining the adsorbent by cooling the calcined product of step (f) at ambient temperature in static condition.

Commercially available adsorbent may used for the preparation of the molecular sieve adsorbent.

In one embodiment of the present invention, the preferred temperature of activation of the molecular sieve adsorbent is about 400°C.

In another embodiment of the present invention, the alkoxides of step (d) used are tetra methyl orthosilicate, tetra ethyl orthosilicate, titanium iso-propoxide, zirconium iso-propoxide and aluminium iso-propoxide.

In still another embodiment, of the present invention the solvent of step (d) used is selected among dry toluene, benzene, cyclohexane and xylene.

In still another embodiment of the present invention, the treatment in the step (d) of the activated molecular sieve is performed bei treating with alkoxide solution or vapours of alkoxide of element M.

In still another embodiment of the present invention, the activated adsorbent in the step (d) is treated with the alkoxide of element M in a dry solvent in the concentration of 0.1 to 1.o % wt / volume for a period in the range of 4 to 8 hours under continuous stirring.

In still another embodiment of the present invention, the activated adsorbent is treated alternatively with the vapours of the alkoxide of element M in the temperature range of 80 to 150°C for a period in the range of 2 to 6 hours.

In still another embodiment of the present invention, the-metal oxide of element M deposition on the microporous solid surface is carried out in a simple liquid phase reaction at ambient temperature and pressure conditions with constant stirring.

In still another embodiment of the present invention, 0.1 to 1.0 wt% of oxide of element M in step (d) is deposited uniformly on the surface of the activated adsorbent.

In still another embodiment of the present invention, the preferable temperature of calcination in step (f) is about 550°C.

In still another embodiment of the present invention, the preferable calcinations time is about 4 hours.

In still another embodiment of the present invention the adsorbent prepared is having adsorption capacity values for methanol in the range from 2.2 to 6.2 wt%, for ethanol in the range from 1.8 to 5.4 wt% and for water in the range from 20.0 to 22.2 wt% and is useful for the dehydration of alcohols and the recovery of alcohol is 99.9%.

We report a novel process to control the pore size or microporous solids like zeolites, which selectively adsorb water from alcohol-water aziotropic mixture. Furthermore this adsorbent displays high thermal and hydrothermal stability.

Microporous solids like zeolites are finding increased applications as adsorbents for separating mixtures of closely related compounds. These have a three dimensional network of basic structural units consisting SiO₄ and AlO₄ tetrahedrons linked to each other by sharing apical oxygen atoms. Silicon and aluminium atoms lie in the centre of the tetrahedral. The resulting alumno-silicate structure, which is generally highly porous, possesses three-dimensional pores the access to which is through molecular sized windows. In a hydrated form, the preferred zeolites are generally represented by the following formula, M_{2/n}O.Al₂O₃.xSiO₂.wH₂O, where M is a cation, which balances the electrovalence of the tetrahedral and is generally referred to as extra framework exchangeable cation, n represents the valancy of the cation and x and w represents the moles of SiO₂ and water respectively.

The attributes which makes the zeolites attractive for separation include, an unusually high thermal and hydrothermal stability, uniform pore structure, easy pore aperture modification and substantial adsorption capacity even at low adsorbate pressures. Furthermore, zeolites can be produced synthetically under relatively moderate hydrothermal conditions.

Structural analysis of the samples was done by X-ray diffraction where in the crystallinity of the zeolites are measured from the intensity of the well-defined peaks. The in situ X-ray powder diffraction measurements at 30°C, 100°C, 200°C, 300°C, 400°C, 500°C, 600°C, 650°C, 700°C, 750°C, 800°C and 850°C shows that the newly developed adsorbent have high thermal stability. X-ray powder diffraction was measured using PHILIPS X'pert MPD system equipped with XRK 900 reaction chamber.

Microporous solid powder with a chemical composition [Na₁₂ (AlO₂)₁₂. (SiO₂) ₁₂.wH₂O] was used as the starting material. X-ray diffraction data showed that the starting material was highly crystalline. A known amount of the powder [Na₁₂ (AlO₂) ₁₂. (SiO₂) ₁₂.wH₂O] was activated at 400°C to remove the water adsorbed and mixed thoroughly with a solution having known amount of alkoxide in 100ml dry solvent, the sample was dried by evaporating solvent under reduced pressure and the alkoxide species deposited on the solids surface was converted into silica by calcinations of the zeolite at 550°C.

Water, methanol and ethanol adsorption were studied gravimetrically after activating the sample at 400°C.

The important inventive steps involved in the present invention are that the molecular sieve adsorbent obtained by the control of the pore mouth of the microporous solids
(i) by the deposition of alkoxide by chemically reacting alkoxide with silanol groups present on the external surface of the activated molecular sieve (zeolite) followed by calcination at 500 - 600°C,
(ii) by liquid phase or vapour phase chemical reaction of alkoxide in moisture free solvent to ensure uniform deposition of silica on the surface of the microporous solid at ambient conditions,
(iii) enhancement of thermal and hydrothermal stability of the adsorbent by alkoxide deposition on the external surface of the solids, and
(iv) to prepare microporous solids based dehydrating adsorbent based on shape /size selectivity by controlling the pore mouths of the solids by depositing inorganic oxides on the external surface.

### Brief description of the table

**Table-1:** The adsorption capacity and selectivity of all the 18 adsorbent samples are enumerated in the

### Examples

The following examples are given by way of illustration and therefore should not be constructed to limit the scope of the present invention.

### EXAMPLE-1

A known amount of microporous solid with chemical composition, [(Na₂O)₆ (Al₂O₃)₆. (SiO₂)₁₂, wH₂O], was activated at 400°C and adsorption measurements were carried out by measuring the adsorption isotherms. Methanol, ethanol and water adsorption capacities are, 18.20%, 15.76% and 22.21% respectively.

### EXAMPLE-2

10.0g of the solid powder [Na₁₂ (AlO₂)₁₂.(SiO₂)₁₂.wH₂O] was activated at 400°C to remove the adsorbed water in the zeolite and stirred with 0.10g tetra methyl orthosilicate in 100ml dry toluene. The sample was dried after 5hrs by evaporating solvent under reduced pressure. The tetra methyl ortho silicate species deposited on the external solid surface was converted into silica by calcinations of the solid at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 6.17%, 5.40% and 21.26% respectively and are given in Table 1.

**TABLE-1**

| **Sample** | **Amount Adsorbed in wt %** | | |
|---|---|---|---|
| | Methanol | Ethanol | Water |
| Example-1 | 18.20 | 15.76 | 22.21 |
| Example-2 | 6.17 | 5.40 | 21.26 |
| Example-3 | 6.18 | 5.39 | 21.33 |
| Example-4 | 6.00 | 5.19 | 21.38 |
| Example-5 | 5.30 | 3.04 | 22.17 |
| Example-6 | 2.40 | 2.08 | 21.15 |
| Example-7 | 2.63 | 1.92 | 21.69 |
| Example-8 | 2.23 | 1.97 | 20.06 |
| Example-9 | 5.37 | 5.02 | 21.97 |
| Example-10 | 2.71 | 2.18 | 21.82 |
| Example-11 | 5.12 | 4.31 | 22.06 |
| Example-12 | 2.51 | 1.84 | 21.87 |
| Example-13 | 2.62 | 1.91 | 22.12 |
| Example-14 | 2.42 | 1.73 | 21.58 |
| Example-15 | 2.52 | 1.93 | 22.15 |
| Example-16 | 2.65 | 1.99 | 21.91 |
| Example-17 | 2.64 | 2.42 | 21.07 |
| Example-18 | 2.57 | 1.82 | 21.43 |

### EXAMPLE-3

10.0g of the solid powder with chemical composition, [Na₁₂ (AlO₂)₁₂.(SiO₂)₁₂.wH₂O] was activated at 400°C to remove the water adsorbed in the solid and stirred with 0.10g tetra ethyl orthosilicate in 100ml dry solvent. The sample was dried after 5 hrs by evaporating solvent under reduced pressure. The tetra ethyl ortho silicate species deposited on the external surface of the solid was converted into silica by calcinations of the zeolite at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 6.17%, 5.39% and 21.33% respectively and are given in Table 1.

### EXAMPLE-4

10.0g of the solid powder with chemical composition, [Na₁₂ (AlO₂)₁₂.(SiO₂)₁₂.wH₂O] was activated at 400°C to remove the water adsorbed in the solid and stirred with 0.15g tetra ethyl orthosilicate in 100ml dry toluene. The sample was dried after 5 hrs by evaporating toluene under reduced pressure. The tetra ethyl ortho silicate species deposited on the external surface of the solid was converted into silica by calcinations of the zeolite at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 6.00%, 5.19% and 21.38% respectively and are given in Table 1.

### EXAMPLE-5

10.0g of the solid powder with chemical composition, [Na₁₂ (AlO₂)₁₂.(SiO₂)₁₂.wH₂O] was activated at 400°C to remove the water adsorbed in the zeolite and stirred with 0.20g tetra ethyl orthosilicate in 100ml dry toluene. The sample was dried after 5 hrs by evaporating toluene under reduced pressure. The tetra ethyl ortho silicate species deposited on the external surface solid was converted into silica by calcinations of the solid at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 5.30%, 5.04% and 22.17% respectively and are given in table 1. 5.0g of the adsorbent in was filled in a column and activated at 400°C by passing 99.5% pure nitrogen gas to remove the adsorbed molecules. The activated adsorbent was cooled to room temperature under inert atmosphere. 25ml ethyl alcohol -water mixture containing 95% ethyl alcohol was passed through the column and the product was analysed by Gas Chromatography and was found that the purity of the product ethanol was 99.9%.

### EXAMPLE-6

10.0g of the solid [Na₁₂ (AlO₂)₁₂.(SiO₂)₁₂.wH₂O] was activated at 400°C to remove the water adsorbed in the zeolite and stirred with 0.25g tetra ethyl orthosilicate in 100ml dry toluene. The sample was dried after 5 hrs by evaporating toluene under reduced pressure. The tetra ethyl ortho silicate species deposited on the external solid surface was converted into silica by calcinations of the solid at 500°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability Methanol, ethanol and water adsorption capacity values are 2.40%, 2.08% and 21.15% respectively and are given in Table 1.

### EXAMPLE-7

10.0g of the solid [Na₁₂ (AlO₂)₁₂.(SiO₂)₁₂.wH₂O] was activated at 400°C to remove the water adsorbed in the zeolite and stirred with 0.30g tetra ethyl orthosilicate in 100ml dry toluene. The sample was dried after 5 hrs by evaporating toluene under reduced pressure. The tetra ethyl ortho silicate species deposited on the external solid surface was converted into silica by calcinations of the zeolite at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 2.63%, 1.92% and 21.69% respectively and are given in table 1. 5.0g of the adsorbent was filled in a column and activated at 400°C by passing 99.5% pure nitrogen gas to remove the adsorbed molecules. The activated adsorbent was cooled to room temperature under inert atmosphere. 25ml ethyl alcohol -water mixture containing 95% ethyl alcohol was passed through the column and the product was analysed by Gas Chromatography and was found that the purity of the product ethanol was 99.9%.

### EXAMPLE-8

10.Og of the solid [Na₁₂ (AlO₂)₁₂.(SiO₂)₁₂.wH₂O] was activated at 400°C to remove the water adsorbed in the zeolite and stirred with 1.00g tetra ethyl orthosilicate in 100ml dry toluene. The sample was dried after 5 hrs by evaporating toluene under reduced pressure. The tetra ethyl ortho silicate species deposited on the external solid surface was converted into silica by calcinations of the zeolite at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 2.23%, 1.97% and 20.06% respectively and are given in table 1. 5.0g of the adsorbent was filled in a column and activated at 450°C by passing 99.5% pure nitrogen gas to remove the adsorbed molecules. The activated adsorbent was cooled to room temperature under inert atmosphere. 25ml methanol -water mixture containing 94% methanol was passed through the column and the product was analysed by Gas Chromatography and was found that the purity of the product methanol was 99.9%.

### EXAMPLE-9

10.0g of the zeolite powder [Na₁₂ (AlO₂)₁₂.(SiO₂)₁₂.wH₂O] was activated at 400°C to remove the water adsorbed in the zeolite and stirred with 0.20g tetra methyl orthosilicate in 100ml dry toluene. The sample was dried after 5 hrs by evaporating toluene under reduced pressure. The tetra methyl ortho silicate species deposited on the zeolite surface was converted into silica by calcinations of the zeolite at 550°C. A known amount of the sample was activated at 400°C under vacuum and adsorption measurements were carried out as described earlier. In situ X-ray powder diffraction measurements at various temperatures up to 850°C shows that the adsorbent has high thermal and hydrothermal stability. Methanol, ethanol and water adsorption capacity values are 5.37%, 5.02% and 21.97% respectively and are given in table 1. 5.0g of the adsorbent in was filled in a column and activated at 400°C by passing 99.5% pure nitrogen gas to remove the adsorbed molecules. The activated adsorbent was cooled to room temperature under inert atmosphere. 25ml ethyl alcohol -water mixture containing 95% ethyl alcohol was passed through the column and the product was analysed by Gas Chromatography and was found that the purity of the product ethanol was 99.9%.

### EXAMPLE-10

10.0g of the solid [Na₁₂(AlO₂)₁₂.(SiO₂)₁₂-wH₂O] was activated at 400°C to remove the water adsorbed in the zeolite and stirred with 0.25g tetra methyl orthosilicate in 100ml dry benzene. The sample was dried after 5 hrs by evaporating benzene under reduced pressure. The tetra methyl ortho silicate species deposited on the solid surface was converted into silica by calcinations of the zeolite at 500°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 2.71%, 2.18% and 21.82% respectively and are given in table 1. 5.0g of the adsorbent was filled in a column and activated at 450°C by passing 99.5% pure nitrogen gas to remove the adsorbed molecules. The activated adsorbent was cooled to room temperature under inert atmosphere. 25ml methanol -water mixture containing 94% methanol was passed through the column and the product was analysed by Gas Chromatography and was found that the purity of the product methanol was 99.9%.

### EXAMPLE-11

10.0g of the solid [Na₁₂(AlO₂)₁₂.(SiO₂)₁₂.wH₂O] was activated at 400°C to remove the

water adsorbed in the zeolite and stirred with 0.20g tetra ethyl orthosilicate in 100ml dry benzene. The sample was dried after 5 hrs by evaporating benzene under reduced pressure. The tetra ethyl ortho silicate species deposited on the solid external surface was converted into silica by calcinations of the zeolite at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 5.12%, 4.31% and 22.06% respectively and are given in table 1. 5.0g of the adsorbent in Example-7 was filled in a column and activated at 400°C by passing 99.5% pure nitrogen gas to remove the adsorbed molecules. The activated adsorbent was cooled to room temperature under inert atmosphere. 25ml ethyl alcohol -water mixture containing 95% ethyl alcohol was passed through the column and the product was analysed by Gas Chromatography and was found that the purity of the product ethanol was 99.9%.

### EXAMPLE-12

10.0g of the solid [Na₁₂(AlO₂)₁₂.(SiO₂)₁₂.wH₂O] was activated at 400°C to remove the water adsorbed in the zeolite and stirred with 0.25g tetra ethyl orthosilicate in 100ml dry cyclohexane. The sample was dried after 5 hrs by evaporating cyclohexane under reduced pressure. The tetra ethyl ortho silicate species deposited on the external solid surface was converted into silica by calcinations of the zeolite at 600°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 2.51%, 1.84% and 21.87% respectively and are given in table 1. 5.0g of the adsorbent was filled in a column and activated at 450°C by passing 99.5% pure nitrogen gas to remove the adsorbed molecules. The activated adsorbent was cooled to room temperature under inert atmosphere. 25ml methanol-water mixture containing 94% methanol was passed through the column and the product was analysed by Gas Chromatography and was found that the purity of the product methanol was 99.9%.

### EXAMPLE-13

10.0g of the solid [Na₁₂(AlO₂)₁₂.(SiO₂)₁₂.wH₂O] was activated at 400°C to remove the water adsorbed in the zeolite and stirred with 0.25g tetra methyl orthosilicate in 100ml dry cyclohexane. The sample was dried after 5 hrs by evaporating cyclohexane under reduced pressure: The tetra methyl ortho silicate species deposited on the external solid surface was converted into silica by calcinations of the zeolite at 550°C. A known amount of the sample was activated at 400°C under vacuum and adsorption measurements were carried out as described earlier. In situ X-ray powder diffraction measurements at various temperatures up to 850°C shows that the adsorbent has high thermal and hydrothermal stability. Methanol, ethanol and water adsorption capacity values are 2.62%, 1.91% and 22.12% respectively and are given in table 1. 5.0g of the adsorbent in was filled in a column and activated at 400°C by passing 99.5% pure nitrogen gas to remove the adsorbed molecules. The activated adsorbent was cooled to room temperature under inert atmosphere. 20ml n-propyl alcohol -water mixture containing 93% n-propyl alcohol was passed through the column and the product was analysed by Gas Chromatography and was found that the purity of the product n-propyl alcohol was 99.9%.

### EXAMPLE-14

10.0g of the solid [Na₁₂(AlO₂)₁₂.(SiO₂)₁₂.wH₂O] was activated at 400°C to remove the water adsorbed in the zeolite and stirred with 0.25g tetra ethyl orthosilicate in 100ml dry xylene. The sample was dried after 5 hrs by evaporating xylene under reduced pressure. The tetra ethyl ortho silicate species deposited on the external solid surface was converted into silica by calcinations of the zeolite at 550°C. A known amount of the sample was activated at 400°C under vacuum and adsorption measurements were carried out as described earlier. *In situ* X-ray powder diffraction measurements at various temperatures up to 850°C shows that the adsorbent has high thermal and hydrothermal stability. Methanol, ethanol and water adsorption capacity values are 2.42%, 1.73% and 21.58% respectively and are given in table 1. 5.0g of the adsorbent was filled in a column and activated at 400°C by passing 99.5% pure nitrogen gas to remove the adsorbed molecules. The activated adsorbent was cooled to room temperature under inert atmosphere. 20ml iso propyl alcohol -water mixture containing 92% iso propyl alcohol was passed through the column and the product was analysed by Gas Chromatography and was found that the purity of the product iso propyl alcohol was 99.9%.

### EXAMPLE-15

10.0g of the solid [Na₁₂(AlO₂)₁₂.(SiO₂)₁₂.wH₂O] was activated at 400°C to remove the water adsorbed in the zeolite and stirred with 0.30g titanium iso-propoxide in 100ml dry xylene. The sample was dried after 5 hrs by evaporating xylene under reduced pressure.

The titanium iso propoxide species deposited on the external solid surface was converted into titania by calcinations of the zeolite at 550°C. A known amount of the sample was activated at 400°C under vacuum and adsorption measurements were carried out as described earlier. Methanol, ethanol and water adsorption capacity values are 2.52%, 1.93% and 22.15% respectively and are given in Table 1.

### EXAMPLE-16

10.0g of the solid powder [Na₁₂(AlO₂)₁₂-(SiO₂)₁₂-wH₂O] was activated at 400°C to remove the water adsorbed in the zeolite and stirred with 0.35g zirconium iso propoxide in 100ml dry xylene. The sample was dried after 5 hrs by evaporating xylene under reduced pressure. The zirconium iso propoxide species deposited on the solid surface was converted into zirconia by calcinations of the zeolite at 550°C. A known amount of the sample was activated at 400°C under vacuum and adsorption measurements were carried out as described earlier. Methanol, ethanol and water adsorption capacity values are 2.65%, 1.99% and 21.91% respectively and are given in Table 1.

### EXAMPLE-17

10.0g of the solid powder [Na₁₂(AlO₂)₁₂.(SiO₂)₁₂.wH₂O] was activated at 400°C to remove the water adsorbed in the solid and stirred with 0.20g aluminium iso propoxide in 100ml dry xylene. The sample was dried after 5 hrs by evaporating xylene under reduced pressure. The aluminium iso propoxide species deposited on the zeolite surface was converted into alumina by calcinations of the zeolite at 500°C. A known amount of the sample was activated at 400°C under vacuum and adsorption measurements were carried out as described earlier. Methanol, ethanol and water adsorption capacity values are 2.69%, 2.42% and 21.07% respectively and are given in Table 1.

### EXAMPLE-18

10.0g of the solid powder [Na₁₂(AlO₂)₁₂.(SiO₂)₁₂.wH₂O] was activated at 400°C to remove the water adsorbed in the zeolite and stirred with 0.25g tetra methyl orthosilicate in 100ml dry xylene. The sample was dried after 5 hrs by evaporating xylene under reduced pressure. The tetra methyl ortho silicate species deposited on the zeolite surface was converted into silica by calcinations of the zeolite at 650°C. A known amount of the sample was activated at 400°C under vacuum and adsorption measurements were carried out as described earlier. In situ X-ray powder diffraction measurements at various temperatures up to 850°C shows that the adsorbent has high thermal and hydrothermal stability. Methanol, ethanol and water adsorption capacity values are 2.57%, 1.82% and. 21.43% respectively and are given in table 1. 5.0g of the adsorbent in was filled in a column and activated at 400°C by passing 99.5% pure nitrogen gas to remove the adsorbed molecules. The activated adsorbent was cooled to room temperature under inert atmosphere. 25ml ethyl alcohol -water mixture containing 95% ethyl alcohol was passed through the column and the product was analysed by Gas Chromatography and was found that the purity of the product ethanol was 99.9%.

The adsorption capacity and selectivity of all the 18 adsorbent samples are enumerated in the Table-1.

### Advantages of the present invention

1. The adsorbent, prepared by the modification of microporous solid selectively adsorbs water over alcohols.
2. The adsorbent is prepared by a simple liquid phase or vapour phase alkoxide deposition.
3. The alkoxide deposition is uniform on the external surface of the microporous solid.
4. The metal alkoxide deposition is carried out at ambient temperature and pressure.
5. The solvent used for the alkoxide deposition can be recovered by distillation method.
6. The adsorbent shows very high thermal and hydrothermal stability.
7. The adsorbent is useful in the commercial drying of alcohols

## Claims

1. A process for the preparation of a molecular sieve adsorbent for adsorptive dehydration of alcohols, said process comprising the steps of:
a) obtaining a molecular sieve adsorbent represented by the chemical formula: (Na₂O)₆·(Al₂O₃)₆·(SiO₂)₁₂·(M_{2/n}O₂)ₓ·wH₂O, where M is the element selected from Si, Al, Zr, and Ti; n is its valance, the values of x varies from 0.001 to 0.1, and w is the number of moles of water;
b) activating the molecular sieve adsorbent at a temperature in the range of 350 to 450°C, to eliminate physically adsorbed water, for a period ranging from 3 to 6 hours;
c) cooling the activated molecular sieve adsorbent under vacuum in the range of 10⁻² to 10⁻⁴ torr;
d) treating the activated adsorbent with an alkoxide of element M in a dry solvent;
e) drying the treated activated adsorbent of step (d) in static conditions at a temperature in the range of 15 to 40 °C;
f) converting the alkoxide of element M deposited on modified adsorbent into metal oxide (M_{2/n}O₂) by calcining the same in a temperature range of 450 to 600°C, for a period ranging from 3 to 8 hours and;
g) obtaining the adsorbent by cooling the calcined product of step (f) at ambient temperature in static condition.

2. A process as claimed in claim 1, wherein the preferred temperature of activation of molecular sieve adsorbent is about 400°C.

3. A process as claimed in claim 1, wherein the alkoxide of step (d) used are tetra methyl orthosilicate, tetra ethyl orthosilicate, titanium iso-propoxide, zirconium iso-propoxide and aluminium iso-propoxide.

4. A process as claimed in claim 1, wherein the solvent of step (d) used are dry toluene, benzene, cyclohexane and xylene.

5. The process as claimed in claim 1, wherein step (d) the treatment of activated molecular sieve is performed by treating with alkoxide solution or vapours of alkoxide of element M.

6. The process as claimed in claim 4, wherein the activated adsorbent in the step (d) is treated with the alkoxide of element M in a dry solvent in the concentration range of 0.1 to 1.0 % wt / volume for a period in the range of 4 to 8 hours under continuous stirring.

7. The process as claimed in claim 4, wherein the activated adsorbent is treated alternatively with the vapours of the alkoxide of element M in the temperature range of 80 to 150°C for a period in the range of 2 to 6 hours.

8. The process as claimed in claim 1, wherein said metal oxide of element M deposition on the microporous solid surface is carried out in a simple liquid phase reaction at ambient temperature and pressure conditions with constant stirring.

9. The process as claimed in claim 5, wherein in step (d) 0.1 to 1.0 wt %of oxide of element M is deposited uniformly on the surface of activated adsorbent.

10. The process as claimed in claim 1, wherein in step (g), the preferable temperature of calcination is about 550°C.

11. A process as claimed in claim 1, wherein the preferable calcinations time is about 4 hours.

12. The process of claim 1, wherein the adsorbent prepared is having adsorption capacity values for methanol in the range from 2.2 to 6.2wt%, for ethanol in the range from 1.8 tao 5.4 wt % and for water in the range from 20.0 to 22.2 wt%, and is useful for the dehydration of alcohols and the recovery of alcohol is 99.9%.

## Patentansprüche

1. Verfahren für die Herstellung eines Molekularsieb-Adsorptionsmittels für die adsorptive Dehydratisierung von Alkoholen, wobei das Verfahren die Schritte umfasst des:
a) Erhaltens eines Molekularsieb-Adsorptionsmittels, das durch die chemische Formel: (Na₂O)₆·Al₂O₃)₆· (SiO₂)₁₂·(M_{2/n}O₂)ₓ·xH₂O dargestellt ist, wobei M das Element ist, das unter Si, Al, Zr und Ti ausgewählt wird; n eine Valenzzahl ist, die Werte von x zwischen 0,001 und 0,1 variieren und w die Anzahl von Molen Wasser ist;
b) Aktivierens des Molekularsieb-Adsorptionsmittels für eine Zeitspanne im Bereich von 3 bis 6 Stunden bei einer Temperatur im Bereich von 350 bis 450 °C, um physikalisch adsorbiertes Wasser zu eliminieren,;
c) Kühlens des aktivierten Molekularsieb-Adsorptionsmittels unter Vakuum im Bereich von 10⁻² bis 10⁻⁴ Torr;
d) Behandelns des aktivierten Adsorptionsmittels mit einem Alkoxid von Element M in einem trockenen Lösungsmittel;
e) Trocknens des behandelten aktivierten Adsorptionsmittels aus Schritt (d) unter statischen Bedingungen bei einer Temperatur im Bereich von 15 bis 40 °C;
f) Umwandelns des Alkoxids von Element M, das auf einem modifizierten Adsorptionsmittel abgesetzt worden ist, in Metalloxid (M_{2/n}O₂) durch Calcinieren desselben in einem Temperaturbereich von 450 bis 600 °C für eine Zeitspanne im Bereich von 3 bis 8 Stunden; und
g) Erhaltens des Adsorptionsmittels durch Kühlen des calcinierten Produkts aus Schritt (f) bei Umgebungstemperatur unter statischen Bedingungen.

2. Verfahren nach Anspruch 1, wobei die bevorzugte Aktivierungstemperatur für Molekularsieb-Adsorptionsmittel etwa 400 °C beträgt

3. Verfahren nach Anspruch 1, wobei das verwendete Alkoxid aus Schritt (d) Tetramethylorthosilicat, Tetraethylorthosilicat, Titanisopropoxid, Zirkoniumisopropoxid und Aluminiumisopropoxid ist.

4. Verfahren nach Anspruch 1, wobei das verwendete Lösungsmittel aus Schritt (d) trockenes Toluol, Benzol, Cyclohexan und Xylol ist.

5. Verfahren nach Anspruch 1, wobei Schritt (d), das Behandeln von aktiviertem Molekularsieb durch Behandeln mit Alkoxidlösung oder Dämpfen von Alkoxid von Element M durchgeführt wird.

6. Verfahren nach Anspruch 4, wobei das aktivierte Adsorptionsmittel in Schritt (d) mit dem Alkoxid von Element M in einem trockenen Lösungsmittel im Konzentrationsbereich von 0,1 bis 1,0 Gew.-%/Volumen für eine Zeitspanne im Bereich von 4 bis 8 Stunden unter kontinuierlichem Rühren behandelt wird.

7. Verfahren nach Anspruch 4, wobei das aktivierte Adsorptionsmittel alternativ mit den Dämpfen des Alkoxids von Element M im Temperaturbereich von 80 bis 150 °C für eine Zeitspanne im Bereich von 2 bis 6 Stunden behandelt wird.

8. Verfahren nach Anspruch 1, wobei das Absetzen des Metalloxids von Element M auf der mikroporösen festen Oberfläche in einer einfachen Flüssigphasenreaktion bei Umgebungstemperatur- und -druckbedingungen unter konstantem Rühren durchgeführt wird.

9. Verfahren nach Anspruch 5, wobei in Schritt (d) 0,1 bis 1,0 Gew.-% Oxid von Element M gleichförmig auf der Oberfläche von aktiviertem Adsorptionsmittel abgesetzt wird.

10. Verfahren nach Anspruch 1, wobei in Schritt (g) die bevorzugte Calcinierungstemperatur etwa 550 °C beträgt.

11. Verfahren nach Anspruch 1, wobei die bevorzugte Caicinierungszeit etwa 4 Stunden beträgt.

12. Verfahren nach Anspruch 1, wobei das hergestellte Adsorptionsmittel Adsorptionskapazitätswerte für Methanol im Bereich von 2,2 bis 6,2 Gew.-%, für Ethanol im Bereich von 1,8 bis 5,4 Ges.-% und für Wasser im Bereich von 20,0 bis 22,2 Ges.-% aufweist und für das die Dehydratisieren von Alkoholen nützlich ist und die Wiedergewinnung von Alkohol 99,9 % beträgt.

## Revendications

1. Procédé de préparation de tamis moléculaire adsorbant pour la déshydratation adsorptive des alcools, ledit procédé comprenant les étapes consistant à :
a) obtenir une matière adsorbante de tamis moléculaire représentée par la formule chimique : (Na₂O)₆•(Al₂O₃)₆•(SiO₂)₁₂•(M_{2/n}O₂)ₓ•wH₂O où M est l'élément choisi parmi Si, Al, Zr, et Ti; n est sa valence, les valeurs de x variant de 0,001 à 0,1 et w est le nombre de moles d'eau ;
b) activer le tamis moléculaire adsorbant à une température située dans la plage allant de 350 à 450 °C, pour éliminer physiquement l'eau adsorbée, pendant une durée allant de 3 à 6 heures ;
c) refroidir le tamis moléculaire adsorbant activé sous vide dans l'intervalle de 10⁻² à 10⁻⁴ torrs ;
d) traiter la matière adsorbante activée avec un alcoxyde de l'élément M dans un solvant sec ;
e) sécher la matière adsorbante activée traitée de l'étape (d) dans des conditions statiques à une température située dans l'intervalle de 15 à 40 °C ;
f) convertir l'alcoxyde de l'élément M déposé sur la matière adsorbante modifiée en oxyde métallique (M_{2/n}O₂) par calcination de celui-ci dans un intervalle de températures allant de 450 à 600 °C, pendant une durée allant de 3 à 8 heures et ;
g) obtenir la matière adsorbante par refroidissement du produit calciné de l'étape (f) à température ambiante dans des conditions statiques.

2. Procédé selon la revendication 1, dans lequel la température préférée d'activation du tamis moléculaire adsorbant est d'environ 400 °C.

3. Procédé selon la revendication 1, dans lequel l'alcoxyde de l'étape (d) utilisé est le tétraméthylorthosilicate, le tétraéthylorthosilicate, l'isopropoxyde de titane, l'isopropoxyde de zirconium et l'isopropoxyde d'aluminium.

4. Procédé selon la revendication 1, dans lequel le solvant de l'étape (d) utilisé est le toluène sec, le benzène, le cyclohexane et le xylène.

5. Procédé selon la revendication 1, dans lequel l'étape (d) de traitement du tamis moléculaire activé est réalisée par traitement avec la solution d'alcoxyde ou des vapeurs d'alcoxyde de l'élément M.

6. Procédé selon la revendication 4, dans lequel la matière adsorbante activée à l'étape (d) est traitée avec l'alcoxyde de l'élément M dans un solvant sec dans l'intervalle de concentration allant de 0,1 à 1,0 % en poids/volume pendant une durée allant de 4 à 8 heures sous agitation continue.

7. Procédé selon la revendication 4, dans lequel la matière adsorbante activée est traitée selon une variante avec les vapeurs d'alcoxyde de l'élément M dans l'intervalle de températures allant de 80 à 150°C pendant une durée allant de 2 à 6 heures.

8. Procédé selon la revendication 1, dans lequel ledit dépôt d'oxyde métallique de l'élément M sur la surface solide microporeuse est réalisé dans une réaction en phase liquide simple dans des conditions de température et de pression ambiantes sous agitation constante.

9. Procédé selon la revendication 5, dans lequel, à l'étape (d), 0,1 à 1,0 % en poids de l'oxyde de l'élément M est déposé uniformément sur la surface de la matière adsorbante activée.

10. Procédé selon la revendication 1, dans lequel, à l'étape (g), la température de calcination préférable est d'environ 550 °C.

11. Procédé selon la revendication 1, dans lequel la durée de calcination préférable est d'environ 4 heures.

12. Procédé selon la revendication 1, dans lequel la matière adsorbante préparée possède des valeurs de capacité d'adsorption pour le méthanol situées dans l'intervalle allant de 2,2 à 6,2 % en poids, pour l'éthanol dans l'intervalle allant de 1,8 à 5,4 % en poids et pour l'eau dans l'intervalle allant de 20,0 à 22,2 % en poids et est utile pour la déshydratation des alcools et la récupération de l'alcool est de 99,9 %.
